# EUROPEAN PATENT APPLICATION

(11) **EP 1 101 456 A1**
(43) Date of publication of application: **23.05.2001**
(21) Application number: 00830749.8
(22) Date of filing: 14.11.2000
(51) Int. Cl.: A61F 2/06

(54) **Vascular implant for the percutaneous treatment of carotid lesions**

(30) Priority: 22.11.1999 IT BO990629
(71) Applicant: Cremonesi, Alberto, 40067 Rastignano (Bologna) (IT); Castriota, Fausto, 40125 Bologna (IT)
(72) Inventor: Cremonesi, Alberto, 40067 Rastignano (Bologna) (IT); Castriota, Fausto, 40125 Bologna (IT)
(74) Representative: Pederzini, Paolo

(57) **Abstract**

A device for a vascular implant in the percutaneous treatment of carotid lesions comprises an expandable endoprosthesis (2) and anti-embolic protection means (3) directly connected to the endoprosthesis (2) and designed to interact with the wall (4) of the blood vessel, to hold the thrombogenic material in place, preventing it from detaching from the wall (4).

## Description

The present invention relates to a device for a vascular implant in the percutaneous treatment of carotid lesions.

Carotid atherosclerosis is the main cause of cerebral ischaemic attacks.

For symptomatic patients, with a carotid stenosis greater than or equal to 70%, the increased incidence of major cerebral attacks (strokes) is 5 - 7% per year.

Various random prospective studies have demonstrated how treatment of critical carotid lesions reduces the risks of subsequent major and/or minor ischaemic attacks both in symptomatic and asymptomatic patients.

At present, carotid artery disease is treated according to two methods, one of which relates to the carotid thromboendarterectomy (TEA) surgical procedure, and the other to percutaneous treatment using the balloon technique (carotid PTA).

Until now, the carotid thromboendarterectomy (TEA) surgical procedure was the elected treatment for obstructive carotid artery disease. However, various retrospective and prospective studies have shown that this method is not risk-free.

The percentages of post-TEA strokes in the NASCET and ECST studies were respectively 5.8% and 7.5%. The incidence of ischaemic attacks was lower in asymptomatic patients treated with TEA in the ACAS group 2.3%.

According to some data which appears in literature, based on a review of many surgical case-reports, the risk of post-TEA neurological damage is around 7.7%.

A separate discussion must be dedicated to patients with associated ischaemic cardiopathy, in which combined endarterectomy and aortic-coronary by pass surgery is correlated with a 4.5 - 7.1% incidence of cerebral ischaemic attacks and a mortality rate varying from 5.5% to 18% depending on the case-report.

Conventional surgery must also take into account the other possible complications, represented mainly by the possibility of damage to cranial nerves, the percentages varying from 7 to 25%, haematomas and/or local infections due to surgical incisions, with a 3.5 - 5% incidence.

The percentage of post-TEA re-stenosis varies greatly, between 2 and 36%.

Clinically relevant re-stenosis occurs in 8 - 10% of cases.

On this subject, it must be emphasised that surgical treatment of re-stenosis is made more serious by an increased peri-operative risk.

Percutaneous treatment using the balloon technique (carotid PTA), although used at carotid level since the far off nineteen eighties, was never approved of, particularly because of the high risk of distal embolisation without the possibility of using suitable cerebral protection.

In fact, whilst percutaneous angioplasty (with or without the use of vascular endoprostheses, or "stents") is certainly a consolidated method, not only at coronary but also at periphery level, its use in the carotid area is still controversial, especially relative to the possible indications, but also regarding both short- and long-term results.

This situation has three main causes:
- specific materials, designed and made for the carotid area are still unavailable for the percutaneous treatment of carotid lesions;
- even the approach and type of technique used are not standardised, making objective comparison of the advantages of one method over others difficult;
- the main complication of a percutaneous angioplasty associated with stent implantation is, even today, a peri-procedural stroke (the frequency varies according to the different case-reports and the types of materials used, but is not below 3%), caused by the detachment of thrombogenic material from the carotid lesion, with subsequent distal embolisation.

The advent of the endovascular stent certainly led many operators to reconsider the possibility of a percutaneous treatment of this vascular area.

There is now much data in literature on many case-reports for patients treated for critical stenosis at carotid level with good immediate results.

The most important case-reports which refer to the work of Roubin, Diethrich, Yadav, Wholey, Mathias and Henry have demonstrated how percutaneous treatment of carotid artery disease is correlated with a risk of cerebral ischaemic attacks and, more generally, a percentage of complications not greater than that for conventional surgery.

The most widely accepted indications for a percutaneous treatment with stents are currently:
- re-stenosis following a previous TEA;
- high carotid stenosis, where surgery necessitates subluxation of the mandible;
- stenosis involving the common carotid artery, which is difficult to reach with a cervical surgery approach;
- stenosis with non-atherosclerotic aetiology (fibromuscular dysplasia, Takayasu's arteritis, radiation therapy).

More controversy is attached to the treatment of very old patients (octogenarians) or those with other serious illnesses, in particular, cases with severe ischaemic cardiopathy (frequently associated with carotid artery disease), where TEA surgery involves an increased risk of peri-operative acute myocardial infarction. On this subject, the initial results of combined treatment with percutaneous angioplasty at both carotid and coronary levels seem very interesting.

Even patients with severe bilateral stenosis or occlusion of an internal carotid artery associated with critical stenosis of the contralateral carotid seem to benefit from a percutaneous treatment during which cerebral ischaemia is limited to the few seconds required to inflate the balloon. In contrast, clamping the carotid during an endarterectomy lasted, on average, for 20 - 40 minutes, in some cases necessitating a surgical shunt.

Some authors maintain that one contraindication for percutaneous treatment is the extensive involvement of the carotid bifurcation, requiring the stent to be positioned across the origin of the external carotid artery. In reality, in many case-reports, the use of self-expanding stents (Wallstents) at the carotid bifurcation only caused significant occlusion or damage to the origin of the external carotid artery in a low percentage of cases, without important clinical implications.

Even in the experience of the Applicants, positioning the stent at the carotid bifurcation level did not cause significant damage and/or occlusion of the homolateral external carotid artery originating from the intrastent portion. Finally, it must be noticed that the absence of a surgical incision reduces the possible local complications and significantly shortens the patient's hospital stay. Most patients treated percutaneously are discharged between 24 and 48 hours after the procedure.

According to Henry, the only real contraindication for a percutaneous treatment is the presence of plaques which are very friable or show evident signs of ulceration. Under such conditions, the risk of a peri-procedural embolism is certainly increased. However, the presence of marked parietal calcification is not an absolute contraindication. For this reason, a correct indication must be based on a careful morphological analysis of the Doppler ultrasound examination.

Another contraindication may be anatomical or pathological features at the level of the aortic arch or the origin of the epiaortic vessels which prevent selective cannulation of the artery to be treated. For such problems, procedures involving direct puncturing of the common carotid artery are described.

The direct experience of the Applicants has demonstrated how, in selected cases, percutaneous dilation of the carotid artery is an effective method which does not expose patients to a risk of complications any greater than that associated with conventional surgery.

It is also believed that the use of low profile coronary angioplasty materials (guides and balloons) reduces the risk of peri-procedural embolic complications even without using coaxial cerebral protection systems: Theron's technique and Kachel's technique.

Moreover, when the degree of stenosis allows it, the possibility of positioning the self-expanding stent without "primary stenting" pre-dilation further reduces the risk of distal embolisation. In such cases, dilation with the balloon is effected exclusively in the intrastent portion.

To reduce the incidence of cerebral attacks correlated with peri-procedural distal embolisation, basically two systems have been proposed until now:
(a) system of distal occlusion using a balloon and aspirating catheter (PercuSurge System - PercuSurge Inc., Sunnyvale, CA), already available on the market; (b) systems of varying complexity, using an umbrella-shaped distal filter able to catch material which comes away from the plaque, but without interrupting the blood flow (not currently commercially available).

However, the above-mentioned systems are characterised by significant objective limitations. Cerebral protection systems based on distal occlusion with a balloon and the removal of thrombogenic material with an aspirating catheter require an inevitable interruption of the carotid flow lasting at least 5 minutes, frequently with clinical and symptomatological implications for patients.

Cerebral protection systems based on umbrella-shaped distal filters able to catch material which comes away from the plaque, without interrupting the carotid blood flow, are technically difficult to create and, at present, are not commercially available.

Whether it consists of an occluding balloon or a filter, the cerebral protection system is mounted on guide wires which, due to their technical characteristics, are rigid and have limited manoeuvrability, making it difficult to use these systems in the case of particularly complex pathologies. Paradoxically, these are the very situations which require an effective protection system.

It is also helpful to remember that the use of such cerebral protection systems is an additional procedure to the PTA/stenting operation.

This consideration leads not only to an increase in difficulty and in procedure times, but also the possibility that the protection system may be the cause of procedural complications.

The aim of the present invention is to make the best possible use of the significant advantages of percutaneous treatment, greatly reducing the risk of embolic events correlated with the detachment of thrombogenic material. This is achieved by a device for a vascular implant in the percutaneous treatment of carotid lesions, which lies within the precise cultural context of contemporary overall treatment of carotid artery disease (of any type and/or in any position) and a simultaneous reduction of the possible peri-procedural embolic complications.

Accordingly, the present invention fulfils this aim using a device for a vascular implant in the percutaneous treatment of carotid lesions, comprising an expandable endoprosthesis, characterised in that it comprises anti-embolic protection means directly attached to the endoprosthesis and designed for insertion between the endoprosthesis and the wall of the blood vessel, in such a way as to hold the thrombogenic material on the wall, preventing it from detaching and so avoiding the complications related to peri-procedural distal embolism.

The device disclosed deals with the problem of treating carotid artery disease and preventing peri-procedural embolic complications from a radically different perspective to that already known: multi-functional self-expanding stents and cerebral protection means are integrated so that they operate in conjunction with each other. Stent positioning and expansion at the site of the lesion are, in themselves, actions which prevent peri-procedural embolic complications.

Moreover, the operator has the added advantage of being able to select the most suitable guide wire for the disease to be treated.

The low profile self-expanding stent (made of steel or nitinol) can guarantee minimum trauma at the site of the carotid lesion.

The anti-embolic mechanism which consists in (partially and/or fully) coating the links of the stent with bio-compatible elastic material, can fix the atherosclerotic plaque to the wall and prevent the passage of pathological material through the links of the stent, which is a primary cause of distal embolism.

Further technical features of the present invention, in accordance with the aforesaid aims, are clearly illustrated in the claims herein, and the advantages of the invention are more clearly shown in the detailed description below, with reference to the accompanying drawings which illustrate a preferred embodiment of the invention without limiting its scope of application and in which:

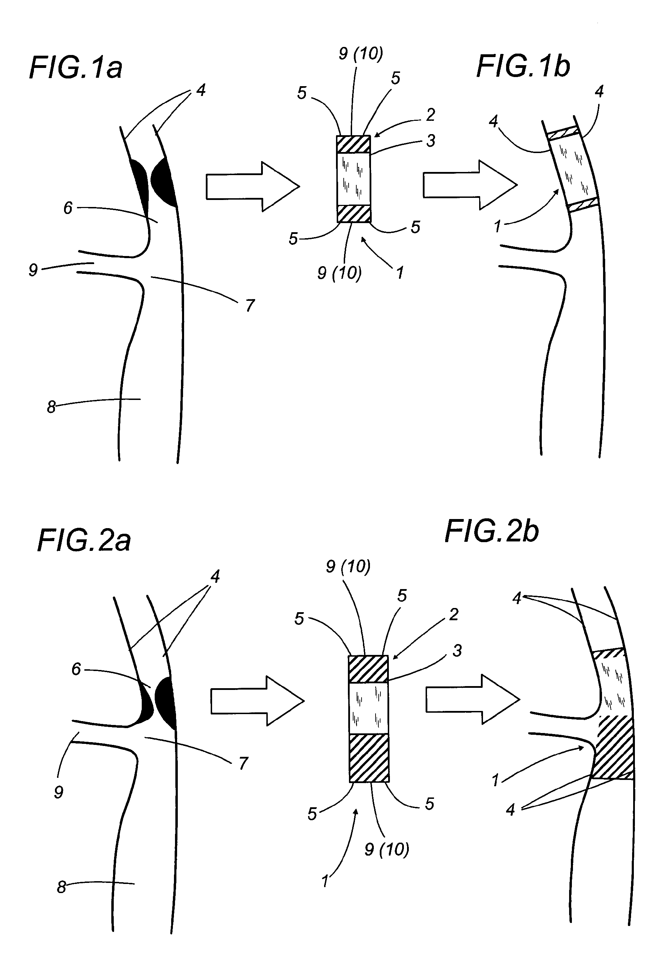

- Figures 1 to 5, in the respective variants a) and b), are schematic illustrations of some important schematic representations of carotid lesions before and after implantation of the device made in accordance with the present invention.

With reference to the accompanying drawings, the numeral 1 indicates as a whole, a device for a vascular implant in the percutaneous treatment of carotid lesions. Said device fulfils the requirement of uniting an endoprosthesis 2 or carotid stent with anti-embolic means 3, attached to the endoprosthesis 2 in such a way as to prevent the complications associated with peri-procedural distal embolism. The aim is to allow the execution of a protected angioplasty and carotid stenting procedure.

More specifically, the endoprosthesis 2 is made of steel or nitinol, that is to say a material which expands automatically at the site of implantation. The anti-embolic protection means basically comprise a filter 3 which is positioned so that it partially covers the endoprosthesis 2. During implantation of the device 1, the filter is inserted between the endoprosthesis 2 and the wall of the blood vessel 4, holding the thrombogenic material in place and preventing it from detaching from the wall of the blood vessel 4.

The filter 3, of which there are many embodiments, all of which may be traced back to a single solution, preferably consists of an expandable membrane made of a bio-absorbable and elastically yielding material wrapped around the endoprosthesis 2.

The filter 3 is tubular and preferably has radiopaque elements 5 which allow the device 1 at the site of implantation to be seen on x-rays. The filter 3 is shorter than the endoprosthesis 2 inside it and is basically between 40% and 90% of the length of the endoprosthesis 2.

This difference in length, that is to say the proportion of endoprosthesis covered by the filter 3, depends on the type and location of the lesions to be treated.

Carotid artery disease may be caused by three basic types of lesions:

Stenosing atherosclerotic plaques: causing obstructions of various size and extension. Ulcerated atherosclerotic plaques: causing erosions of the wall of the blood vessel, with frequent disorganised thrombotic formations adhering to the surface of the plaque and/or with intimal flaps which project into the lumen of the blood vessel. Parietal dissection (spontaneous, post-traumatic or iatrogenic): intimal wall ruptures, with intimal flaps which project into the lumen of the blood vessel and frequent cases of double endovascular lumina.

The above-mentioned types of lesions may coexist within the same patient, involving different sectors of the carotid.

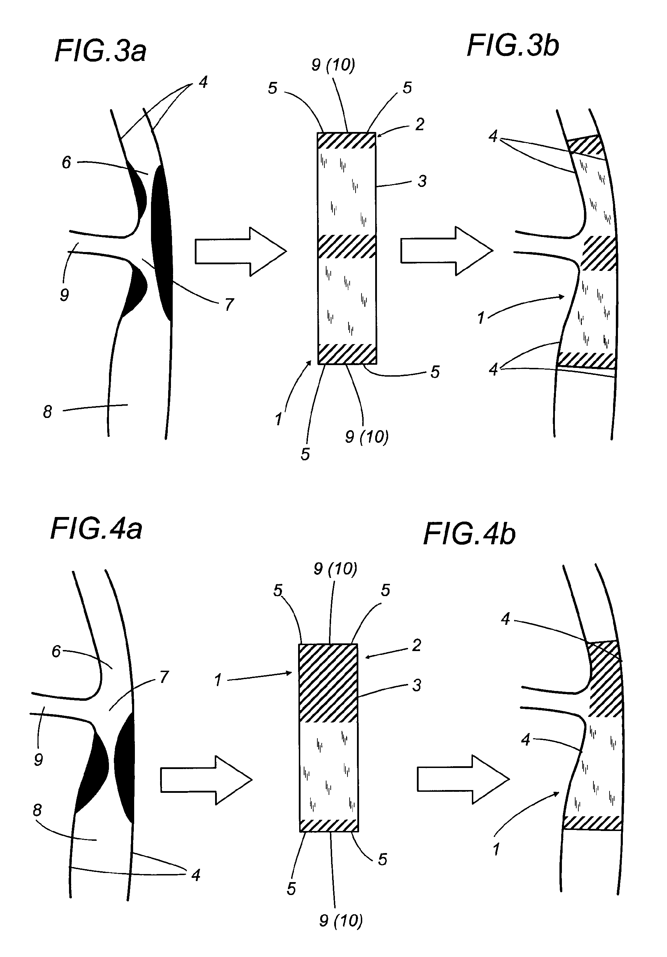

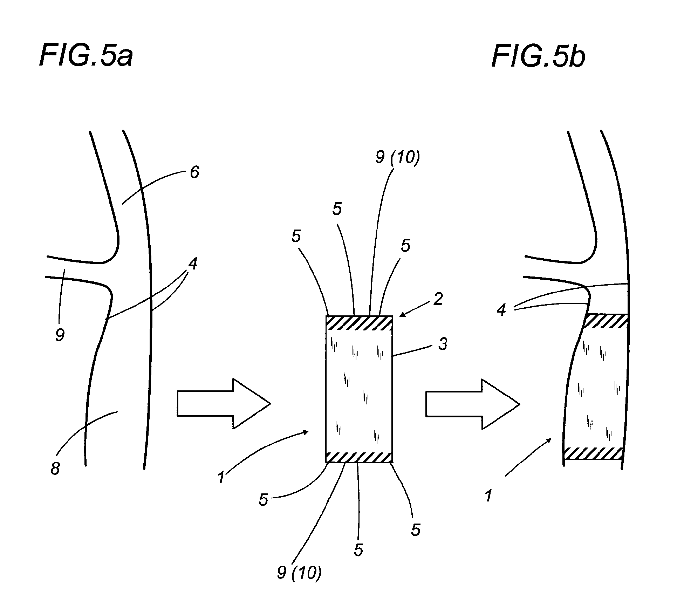

From an anatomical viewpoint, carotid lesions may be divided as follows:
- localised lesions of the internal carotid artery 6, without involving the carotid bifurcation 7 (see Figure 1a);
- localised lesions of the internal carotid artery 6, at the ostium, with minimal involvement of the carotid bifurcation 7 (see Figure 2a);
- extensive lesions of the internal carotid artery 6, at the ostium, with severe involvement of the carotid bifurcation 7 (see Figure 3a);
- lesions of the carotid bifurcation 7 with minimal involvement of the internal carotid artery 6 (see Figure 4a);
- lesions of the common carotid artery 8 (see Figure 5a).

The design of the device 1 disclosed provides for the development of five indicative embodiments of the device 1, which do not limit the scope of the present invention, designed to provide a complete solution for the treatment of any type of carotid lesion, in any position, also maintaining the patency of the external carotid artery 9.

A first embodiment of such a device, labelled A and indicated in localised lesions of the internal carotid artery 6 without involvement of the carotid bifurcation 7 is illustrated in Figure 1b, before and after insertion in the carotid.

A device 1 designed for such use would, by way of example, have the following features:
- Length of the expanded endoprosthesis 2 (in mm): 30, 40.
- Length of the section(s) covered by the filter 3 (in mm): 20, 27.
- Diameter of the expanded endoprosthesis 2 (in mm): 7, 9.

The filter 3 is fitted on the endoprosthesis 2 at the centre of the latter's length.

A second embodiment, labelled B and indicated in localised lesions of the internal carotid artery 6, at the ostium, with minimal involvement of the carotid bifurcation 7, is illustrated in Figure 2b, before and after insertion in the carotid.

Its dimensions are as follows:
- Length of the expanded endoprosthesis (in mm): 40, 50.
- Length of the section(s) covered by the filter (in mm): 20, 27.
- Diameter of the expanded stent (in mm): 7, 9, 11.

The filter 3 is located on the endoprosthesis 2 in a position which is offset relative to the centre line on the length of the endoprosthesis.

A third embodiment of the device 1, labelled C and indicated in extensive lesions of the internal carotid artery 6, at the ostium and with severe involvement of the carotid bifurcation 7, is illustrated in Figure 3b, before and after insertion in the implant site.

Its dimensions are as follows:
- Length of the expanded stent (in mm): 50, 60.
- Length of the section(s) covered (in mm): 20, 25.
- Distance between covered sections (to allow external carotid artery patency): 7.
- Diameter of the expanded stent (in mm): 8, 10, 12.

The filter 3 is divided into two sections of substantially equal length, positioned at the internal carotid artery 6 and the common carotid artery 8.

A fourth embodiment of the device 1, labelled D and indicated in lesions of the carotid bifurcation 7 with minimal involvement of the internal carotid artery 6, is illustrated in Figure 4b, before and after insertion at the implant site.

Its dimensions are as follows:
- Length of the expanded stent (in mm): 40.
- Length of the section(s) covered (in mm): 27.
- Diameter of the expanded stent (in mm): 8, 10, 12.

The filter 3 is fitted on the endoprosthesis 2 in a position which is offset relative to the centre line on the length of the endoprosthesis 2.

A fifth embodiment of the device 1, labelled E, indicated in lesions of the common carotid artery 8, is illustrated in Figure 5b, before and after insertion in the implant site.

Its dimensions are as follows:
- Length of the expanded stent (in mm): 40, 50, 60.
- Length of the section(s) covered (in mm): 32, 42, 52.
- Diameter of the expanded stent (in mm): 10, 12.

The filter 3 is fitted on the endoprosthesis 2 in a position which is centred relative to the centre line on the length of the endoprosthesis 2.

The bio-material of which the filter 3 is made must have proven bio-compatibility features and must be elastic enough to prevent it counteracting the force exerted by the stent (radial force) on the wall of the blood vessel 4.

In order to have an anti-embolic action, the material must guarantee perfect adhesion to and covering of the stent 2 links, even when the stent is fully expanded, to prevent potentially dangerous ruptures.

The thickness of the membrane or film must be minimal, so that, once applied on the stent 2, it does not compromise the "crossing profile" features of the stent 2.

The extension of the anti-embolic covering over the stent 2 must show up on x-rays (radiopaque markers on the proximal 9 and distal 10 edges of the cover), to allow suitable system positioning.

The present invention may have obvious industrial applications and may be subject to numerous variations, all encompassed by the original design concept. Moreover, all parts may be substituted with technically equivalent elements.

## Claims

1. A device for a vascular implant, in particular in the percutaneous treatment of carotid lesions, comprising an expandable endoprosthesis (2), characterised in that it comprises anti-embolic protection means (3), the latter being directly attached to the endoprosthesis (2) and designed to interact with the wall (4) of the blood vessel, holding the thrombogenic material in place and preventing its detachment from the wall (4).

2. The device according to claim 1, characterised in that the anti-embolic protection means comprise a filter (3) which is positioned so that it at least partially covers the endoprosthesis (2) and which, during implantation of the device (1), is inserted between the endoprosthesis (2) and the wall (4) of the blood vessel, holding the thrombogenic material in place and preventing its detachment from the wall (4) of the blood vessel.

3. The device according to claim 1 or 2, characterised in that the endoprosthesis (2) is self-expanding.

4. The device according to claim 2, characterised in that the filter (3) consists of an expandable membrane which is wrapped around the endoprosthesis (2).

5. The device according to claim 2, characterised in that the filter (3) consists of a membrane made of an elastically yielding material.

6. The device according to any of the foregoing claims, characterised in that the membrane is made of a bio-absorbable material.

7. The device according to any of the foregoing claims from 2 to 6, characterised in that the filter (3) is tubular in shape.

8. The device according to any of the foregoing claims, characterised in that the filter (3) is shorter than the endoprosthesis (2) inside it.

9. The device according to claim 8, characterised in that the length of the filter (3) is between 40% and 90% of the length of the endoprosthesis (2).

10. The device according to any of the foregoing claims, characterised in that it comprises radiopaque elements (5), being attached to the filter (3) to allow the device to show up on x-rays at the site of the implant.

11. The device according to claim 8 or 9, characterised in that, in application in localised lesions of the internal carotid artery (6) without involvement of the carotid bifurcation (7), the length of the filter (3) is around 67% of the total length of the endoprosthesis (2).

12. The device according to claim 11, characterised in that the filter (3) is fitted on the endoprosthesis (2) in a position which is centred relative to the length of the latter.

13. The device according to claim 8 or 9, characterised in that, in application in localised lesions of the internal carotid artery (6) at the ostium with minimal involvement of the carotid bifurcation (7), the length of the filter (3) is around 50% of the total length of the endoprosthesis (2).

14. The device according to claim 13, characterised in that the filter (3) is fitted on the endoprosthesis (2) in a position which is offset relative to the centre line on the length of the endoprosthesis (2).

15. The device according to claim 8 or 9, characterised in that, in application in extensive lesions of the internal carotid artery (6) at the ostium with severe involvement of the carotid bifurcation (7), the length of the filter (3) is around 40% of the total length of the endoprosthesis (2).

16. The device according to claim 15, characterised in that the filter (3) is divided into two sections, having substantially equal length and being positioned respectively at the internal carotid artery (6) and at the common carotid artery (8).

17. The device according to claim 8 or 9, characterised in that, in application to lesions of the carotid bifurcation (7) with minimal involvement of the internal carotid artery (6), the length of the filter (3) is substantially around 68% of the total length of the endoprosthesis (2).

18. The device according to claim 17, characterised in that the filter (3) is fitted on the endoprosthesis (2) in a position which is offset relative to the centre line on the length of the endoprosthesis (2).

19. The device according to claim 8 or 9, characterised in that, in application to lesions of the common carotid artery (8), the length of the filter (3) is substantially around 80% of the total length of the endoprosthesis (2).

20. The device according to claim 19, characterised in that the filter (3) is fitted on the endoprosthesis (2) in a position which is centred relative to the centre line on the length of the endoprosthesis (2).
